# NEW EUROPEAN PATENT SPECIFICATION

(11) **EP 2 082 054 B2**
(45) Date of publication and mention of the opposition decision: **03.10.2018**
(45) Mention of the grant of the patent: 16.05.2012
(21) Application number: 07875026.2
(22) Date of filing: 13.11.2007
(51) Int. Cl.: C12P 19/02, C12P 19/12

(54) **METHOD FOR IMPROVING THE YIELD OF CELLULOSE CONVERSION PROCESSES**
VERFAHREN ZUR VERBESSERUNG DER AUSBEUTE BEI CELLULOSEUMWANDLUNGSPROZESSEN
PROCÉDÉ D'AMÉLIORATION DU RENDEMENT DE PROCÉDÉS DE CONVERSION DE CELLULOSE

(30) Priority: 13.11.2006 US 858579 P
(43) Date of publication of application: 29.07.2009
(73) Proprietor: Danisco US Inc., Palo Alto, CA 94304 (US)
(72) Inventor: KELEMEN, Bradley, Palo Alto, California 94304 (US); LARENAS, Edmund A., Palo Alto, California 94304 (US); MITCHINSON, Colin, Palo Alto, California 94304 (US)
(74) Representative: Kiddle, Simon John
(86) International application number: PCT/US2007/023732
(87) International publication number: WO 2008/147396

(56) References cited:
- EP-A1- 1 225 227
- WO-A-2005/001065
- WO-A1-92/10581
- WO-A2-2005/074647
- WO-A2-2005/074656
- WO-A2-2005/100582
- TOLAN: "Iogen's process for producing ethanol from cellulosic biomass" CLEAN TECHNOLOGY AND ENVIRONMENTAL POLICY, vol. 3, 2002, pages 339-345, XP002510177
- ORTEGA ET AL: "Kinetics of cellulose saccharification by Trichoderma reesei cellulases" INTERNATIONAL BIODETERIORATION & BIODEGRADATION, vol. 47, 2001, pages 7-14, XP002975525
- ADEN ET AL: "Lignocellulosic biomass to ethanol process design and economics utilizing co-current dilute acid prehydrolysis and enzymatic hydrolysis for corn stover" 2002, page 1,28,29,34, XP002510398 Retrieved from the Internet: URL:www.nrel.gov/docs/fy02osti/32438.pdf> [retrieved on 2009-01-14]
- JOHNSON ET AL: "Saccharification of complex cellulosic substrates by the cellulase system from Clostridium thermocellum" APPLIED AND ENVIRONMENTAL MICROBIOLOGY, vol. 43, 1982, pages 1125-1132, XP002510500
- VIIKARI ET AL: "Thermostable enzymes in lignocellulose hydrolysis" ADVANCES IN BIOCHEMICAL ENGINEERING/BIOTECHNOLOGY, vol. 108, 23 June 2007 (2007-06-23), pages 121-145, XP002510178
- ENARI ET AL: 'PURIFICATION OF TRICHODERMA REESEI AND ASPERGILLUS NIGER [BETA]-GLUCOSIDASE' JOURNAL OF APPLIED BIOCHEMISTRY vol. 3, 1981, ACADEMIC PRESS, LONDON, GB, pages 157 - 163, XP002564087
- DURAND ET AL: 'BIOCHEMISTRY AND GENETICS OF CELLULOSE DEGRADATION' ACADEMIC PRESS 1988, pages 135 - 151
- JANUSZ SZCZODRAK: 'THE ENZYMATIC HYDROLYSIS AND FERMENTATION OF PRETREATED WHEAT STRAW TO ETHANOL' BIOTECHNOLOGY AND BIOENGENEERING vol. 32, no. 6, 1988, WILEY, US, pages 771 - 776, XP001320289
- BRINK: 'DILUTE NITRIC ACID PRETREATMENT AND ANZYMATIC HYDROLYSIS OF LIGNOCELLULOSIC MATERIALS' UNIVERSITY OF CALIFORNIA/ FOREST PRODUCTS LABORATORY 1995, pages 1 - 136
- DESAI ET AL: 'SUBSTRATE REACTIVITY AS A FUNCTION OF THE EXTENT OF REACTION IN THE ENZYMATIC HYDROLYSIS OF LIGNOCELLULOSE' BIOTECHNOLOGY AND BIOENGINEERING vol. 56, no. 6, 20 December 1997, WILEY, US, pages 650 - 655, XP002676966
- BROWN ET AL: 'ENZYMATIC SACCHARIFICATION OF LIGNOCELLULOSIC BIOMASS//NREL/ETHANOL PROJECT' LAP-009 1996, pages 1 - 8
- CELLUCLAST 1.5L, NOVO NORDISK, ENZYME PROCESS DIVISION 1993, pages 1 - 2
- SPEZYME CP, GENECOR CELLULASE ENZYME 1996, pages 1 - 2
- GODLIVING MTUIET AL: 'BIOCONVERSION OF LIGNOCELLULOSIC WASTE FROM SELECTED DUMPING SITES IN DAR ES SALAAM, TANZANIA' BIODEGRADATION vol. 16, no. 6, 01 December 2005, KLUWER ACADEMIC PUBLISHERS, DO, pages 493 - 499, XP019231934
- DALE ET AL: 'A PILOT PLANT SCALE REACTOR/SEPARATOR FOR ETHANOL FROM CELLULOSICS' ERIP/DOE QUARTERLY REPORT # 3&4 1998, pages 1 - 13
- MOSIER N. ET AL: 'FEATURES OF PROMISING TECHNOLOGIES FOR PRETREATMENT OF LIGNOCELLULOSIC BIOMASS' BIORESOURCE TECHNOLOGY vol. 96, no. 6, 2005, ELSEVIER, AMSTERDAM, NL, pages 673 - 686, XP004664610
- ZHANG ET AL BIOCHEMISTRY vol. 34, 1995, pages 3386 - 3395
- COPELAND: 'ENZYMES: A PRACTICAL INTRODUCTION TO STRUCTURE, MECHANISM AND DATA ANALYSIS.' CHAPTER/WILEY-VCH 2000, pages 247 - 251
- LATIF ET AL BIORESOURCE TECHNOLOGY vol. 50, 1994, pages 107 - 111
- REESE M.G. ET AL: 'IMPROVED SPLICE SITE DETECTION IN GENIE' BIOTECHNOLOGY AND BIOENGINEERING vol. 22, no. 2, 01 February 1980, pages 323 - 335, XP055058050
- TABKA M.G. ET AL: 'ENZYMATIC SACCHARIFICATION OF WHEAT STRAW FOR BIOETHANOL PRODUCTION BY A COMBINED CELLULASE XYLANASE AND FERULOYL ESTERASE TREATMENT' ENZYME AND MICROBIAL TECHNOLOGY vol. 39, no. 4, 01 August 2006, STONEHAM, MA, US, pages 897 - 902, XP002511088
- ORTEGA N. ET AL: 'KINETICS OF CELLULOSE SACCHARIFICATION BY TRICHODERMA REESEI CELLULASES' INTERNATIONAL BIODETERIORATION & BIODEGRADATION vol. 47, 2001, ELSEVIER, AMSTERDAM, NL, pages 7 - 14, XP002975525
- NAGIEB ET AL JOURNAL OF APPLIED POLYMER SCIENCE vol. 30, 1985, pages 4653 - 4658
- YE SUN ET AL: 'HYDROLYSIS OF LIGNOCELLULOSIC MATERIALS FOR ETHANOL PRODUCTION: A REVIEW' BIORESOURCE TECHNOLOGY vol. 83, no. 1, 01 January 2002, ELSEVIER, AMSTERDAM, NL, pages 1 - 11, XP002670852
- MURRAY P. ET AL: 'EXPRESSION IN TRICHODERMA REESEI AND CHARACTERISATION OF A THERMOSTABLE FAMILY 3@B-GLUCOSIDASE FROM THE MODERATELY THERMOPHILIC FUNGUS TALAROMYCES EMERSONII' PROTEIN EXPRESSION AND PURIFICAT vol. 38, no. 2, 01 December 2004, ACADEMIC PRESS, SAN DIEGO, CA, pages 248 - 257, XP004649879
- LYND L.R. ET AL: 'MICROBIAL CELLULOSE UTILIZATION: FUNDAMENTALS AND BIOTECHNOLOGY' MICROBIOLOGY AND MOLECULAR BIOLOGY REVIEWS vol. 66, no. 3, 01 September 2002, AMERICAN SOCIETY FOR MICROBIOLOGY, US, pages 506 - 577, XP001120774
- DANIEL J. SCHELL ET AL: 'DILUTE-SULFURIC ACID PRETREATMENT OF CORN STOVER IN PILOT-SCALE REACTOR' APPLIED BIOCHEMISTRY AND BIOTECHNOLOGY vol. 105, no. 1-3, 01 January 2003, HUMANA PRESS INC, NEW YORK, pages 69 - 85, XP002670853
- SHENG ZHANG ET AL: 'SITE-DIRECTED MUTATION OF NONCATALYTIC RESIDUES OF THERMOBIFIDA FUSCA EXOCELLULASE CEL6B' EUROPEAN JOURNAL OF BIOCHEMI vol. 267, 01 January 2000, WILEY-BLACKWELL PUBLISHING LTD, GB, pages 3101 - 3115, XP008137067
- WALKER L.P. ET AL: 'ENGINEERING CELLULASE MIXTURES BY VARYING THE MOLE FRACTION OF THERMOMONOSPORA FUSCA E-5 AND E-3, TRICHODERMA REESEI CBHI, AND CALDOCELLUM SACCHAROLYTICUM BETA-GLUCOSIDASE' BIOTECHNOLOGY AND BIOENGINEERING vol. 42, no. 9, 01 January 1993, WILEY, US, pages 1019 - 1028, XP002498060
- STERNBERG D. ET AL: 'BETA-GLUCOSIDASE: MICROBIAL PRODUCTION AND EFFECT ON ENZYMATIC HYDROLYSIS OF CELLULOSE' CANADIAN JOURNAL OF MICROBIOLOGY vol. 23, no. 2, 01 February 1977, NRC RESEARCH PRESS, CA, pages 139 - 147, XP002564234
- SZCZODRAK J.: 'THE USE OF CELLULASES FROM A BETA-GLUCOSIDASE-HYPERPRODUCING MUTANT OF TRICHODERMA REESEI IN SIMULTANEOUS SACCHARIFICATION AND FERMENTATION OF WHEAT STRAW' BIOTECHNOLOGY AND BIOENGINEERING vol. 33, no. 9, 05 April 1989, WILEY, US, pages 1112 - 1116, XP002564514
- PETERSON R. ET AL: 'TRICHODERMA REESEI RUT-C30 - THIRTY YEARS OF STRAIN IMPROVEMENT' MICROBIOLOGY vol. 158, no. 1, 01 January 2012, SOCIETY FOR GENERAL MICROBIOLOGY, pages 58 - 68, XP055105400
- HTTP://WWW.LGCSTANDARDS-ATCC.ORG/PRODUCTS/A LL/26921.ASPX?GEO_COUNTRY=DE, 2014(?)

## Description

### 3. FIELD

The present teaching relates to methods for improving the yield of desirable sugars in the enzymatic conversion of cellulosic materials.

### 4. BACKGROUND

The production of sugars from cellulosic materials has been known for some time, as has the subsequent fermentation and distillation of these sugars into ethanol. Much of the prior development occurred around the time of World War II when fuels were at a premium in such countries as Germany, Japan and the Soviet Union. These early processes were primarily directed to acid hydrolysis but were fairly complex in their engineering and design and were very sensitive to small variations in process variables, such as temperature, pressure and acid concentrations. A comprehensive discussion of these early processes is presented in "Production of Sugars From Wood Using High-pressure Hydrogen Chloride", Biotechnology and Bioengineering, Volume XXV, at 2757-2773 (1983),

The abundant supply of petroleum in the period from World War II through the early 1970s slowed ethanol conversion research. However, due to the oil crisis of 1973, researchers increased their efforts to develop processes for the utilization of wood and agricultural byproducts for the production of ethanol as an alternate energy source. This research was especially important for development of ethanol as a gasoline additive to reduce the dependency of the United States upon foreign oil production, to increase the octane rating of fuels, and to reduce exhaust pollutants as an environmental measure.

Concurrently with the "oil crisis," as it became known, the Environmental Protection Agency of the United States promulgated regulations requiring the reduction of lead additives in an effort to reduce air pollution. Insofar as ethanol is virtually a replacement of lead, some refineries have selected ethanol as the substitute, especially since it can easily be introduced into a refinery's operation without costly capital equipment investment.

In addition to improving the high pressure and high temperature gas saccharification processes developed decades ago, current research is directed primarily at enzymatic conversion processes. These processes employ enzymes from a variety of organisms, such as mesophilic and thermophilic fungi, yeast and bacteria, which degrade the cellulose into fermentable sugars. Uncertainty still remains with these processes and their ability to be scaled up for commercialization as well as their inefficient rates of ethanol production.

Cellulose and hemicellulose are the most abundant plant materials produced by photosynthesis. They can be degraded for use as an energy source by numerous microorganisms, including bacteria, yeast and fungi, which produce extracellular enzymes capable of hydrolysis of the polymeric substrates to monomeric sugars (Aro et al., 2001). Organisms are often restrictive with regard to which sugars they use and this dictates which sugars are best to produce during conversion. As the limits of non-renewable resources approach, the potential of cellulose to become a major renewable energy resource is enormous (Krishna et al., 2001). The effective utilization of cellulose through biological processes is one approach to overcoming the shortage of foods, feeds, and fuels (Ohmiya et al., 1997).

Cellulases are enzymes that hydrolyze cellulose (beta-1,4-glucan or beta D-glucosidic linkages) resulting in the formation of glucose, cellobiose, cellooligosaccharides, and the like. Cellulases have been traditionally divided into three major classes: endoglucanases (EC 3.2.1.4) ("EG"), exoglucanases or cellobiohydrolases (EC 3.2.1.91) ("CBH") and beta-glucosidases ([beta] -D-glucoside glucohydrolase; EC 3.2.1.21) ("BG"). (Knowles et al., 1987 and Shulein, 1988). Endoglucanases act mainly on the amorphous parts of the cellulose fiber, whereas cellobiohydrolases are also able to degrade crystalline cellulose.

Cellulases have also been shown to be useful in degradation of cellulose biomass to ethanol (wherein the cellulases degrade cellulose to glucose and yeast or other microbes further ferment the glucose into ethanol), in the treatment of mechanical pulp (Pere et al., 1996), for use as a feed additive (WO 91/04673) and in grain wet milling. Separate saccharification and fermentation is a process whereby cellulose present in biomass, e.g., corn stover, is converted to glucose and subsequently yeast strains convert glucose into ethanol. Simultaneous saccharification and fermentation is a process whereby cellulose present in biomass, e.g., corn stover, is converted to glucose and, at the same time and in the same reactor, yeast strains convert glucose into ethanol. Ethanol production from readily available sources of cellulose provides a stable, renewable fuel source.

Cellulases are known to be produced by a large number of bacteria, yeast and fungi. Certain fungi produce a complete cellulase system (i.e., a whole cellulase) capable of degrading crystalline forms of cellulose. In order to efficiently convert crystalline cellulose to glucose the complete cellulase system comprising components from each of the CBH, EG and BG classifications is required, with isolated components less effective in hydrolyzing crystalline cellulose (Filho et al., 1996). In particular, the combination of EG-type cellulases and CBH-type cellulases interact to more efficiently degrade cellulose than either enzyme used alone (Wood, 1985; Baker et al., 1994; and Nieves et al., 1995).

Additionally, cellulases are known in the art to be useful in the treatment of textiles for the purposes of enhancing the cleaning ability of detergent compositions, for use as a softening agent, for improving the feel and appearance of cotton fabrics, and the like (Kumar et al., 1997). Cellulase-containing detergent compositions with improved cleaning performance (US Pat. No. 4,435,307; GB App. Nos. 2,095,275 and 2,094,826) and for use in the treatment of fabric to improve the feel and appearance of the textile (US Pat. Nos. 5,648,263, 5,691,178, and 5,776,757, and GB App. No. 1,358,599), have been described.

Hence, cellulases produced in fungi and bacteria have received significant attention. In particular, fermentation of *Trichoderma spp.* (e.g., *Trichoderma longibrachiatum* or *Trichoderma reesei*) has been shown to produce a complete cellulase system capable of degrading crystalline forms of cellulose. Over the years, *Trichoderma* cellulase production has been improved by classical mutagenesis, screening, selection and development of highly refined, large scale inexpensive fermentation conditions. While the multi-component cellulase system of *Trichoderma spp.* is able to hydrolyze cellulose to glucose, there are cellulases from other microorganisms, particularly bacterial strains, with different properties for efficient cellulose hydrolysis, and it would be advantageous to express these proteins in a filamentous fungus for industrial scale cellulase production. However, the results of many studies demonstrate that the yield of bacterial enzymes from filamentous fungi is low (Jeeves et al., 1991).

Soluble sugars, such as glucose and cellobiose, have a multitude of uses in industry for the production of chemicals and biological products. The optimization of cellulose hydrolysis allows for the use of lower quantities of enzyme and improved cost effectiveness for the production of soluble sugars. Despite the development of numerous approaches, there remains a need in the art for improving the yield of soluble sugars obtained from cellulosic materials.
Tolan (Clean Tech Environ Policy, 2002, 339-345) discloses an overview of a process for producing ethanol from cellulosic biomass, including using cellulase in a cellulase hydrolysis reaction. The cellulosic material is processed in the hydrolysis reaction for 5 to 7 days at 50°C.
Vlikara et al. (Advances in Biochem. Engin./Biotechnol, 2007, 121-145) discloses a comparison of thermostable enzymes and commercial cellulases in lignocellulose hydrolysis.
WO 2005/001065 discloses variant *Humicola grisea* CBH1.1 having cellobiohydralase activity.

### 5. SUMMARY

According to the invention there is provided a method according to the appended claim 1. The present teachings provide methods for increasing the yield of glucose from the enzymatic saccharification of cellulosic starting materials by incubating a cellulosic substrate or a pretreated cellulosic substrate with a cellulase at a temperature at or about the thermal denaturation temperature of the cellulase.

Also disclosed are methods for converting a cellulosic material to glucose by combining a cellulosic material with a cellulase incubating the cellulosic material and cellulase combination at a temperature greater than about 38 °C to cause a hydrolysis reaction to convert at least 20% of said cellulosic material to soluble sugars, wherein the fraction of glucose is at least 0.75 relative to the soluble sugars.

The cellulases are whole cellulases, produced by microorganisms from, species *Trichoderma reesei*

These and other features of the present teachings are provided herein.

### 6. BRIEF DESCRIPTION OF THE DRAWINGS

The skilled artisan will understand that the drawings are for illustration purposes only and are not intended to limit the scope of the present teachings in anyway. Figures 1 to 10 and the data corresponding to 53°C in Figures 11 and 12 are comparative examples.

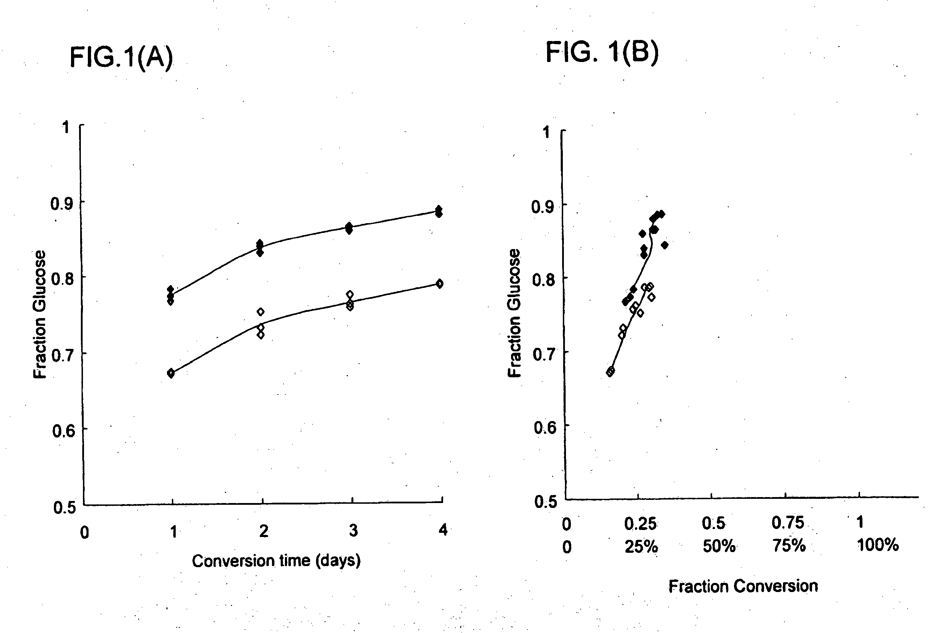

FIGS. 1A-B show the conversion of dilute acid treated corn stover to soluble sugars by 3.3 mg/g of whole cellulase from *Trichoderma reesei* over-expressing beta-glucosidase, at 38 °C (open symbols) and 53 °C (closed symbols).

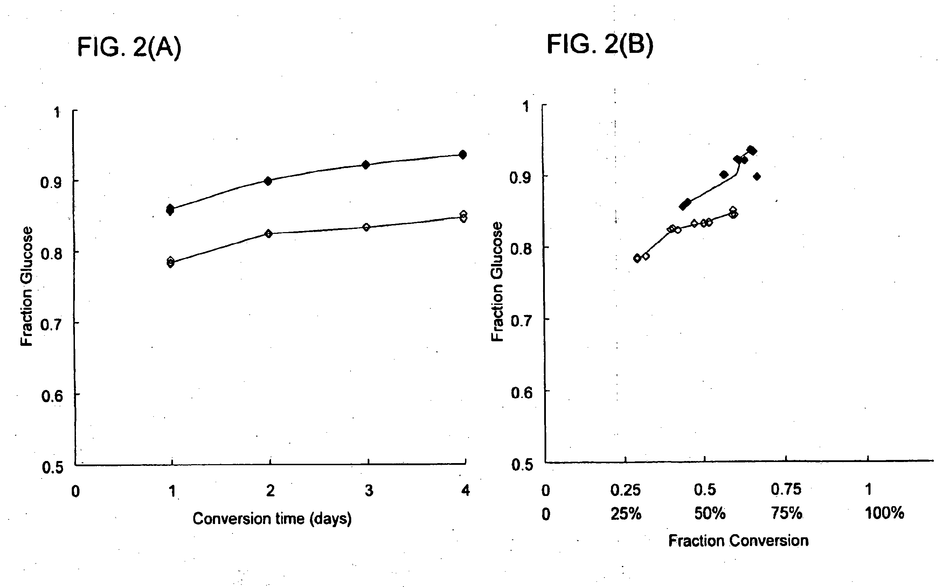

FIGS. 2A-B show the conversion of dilute acid treated corn stover to soluble sugars by 12 mg/g of whole cellulase from *Trichoderma reesei* over-expressing beta-glucosidase, at 38 °C (open symbols) and 53 °C (closed symbols).

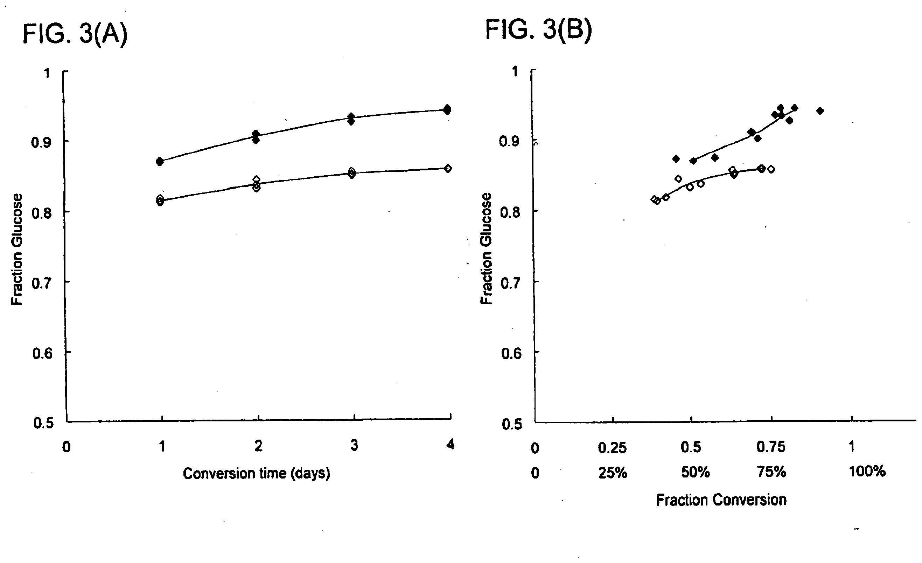

FIGS. 3A-B show the conversion of dilute acid treated corn stover to soluble sugars by 18 mg/g of whole cellulase from *Trichoderma reesei* over-expressing beta-glucosidase 1, 38 °C (open symbols) and 53 °C (closed symbols).

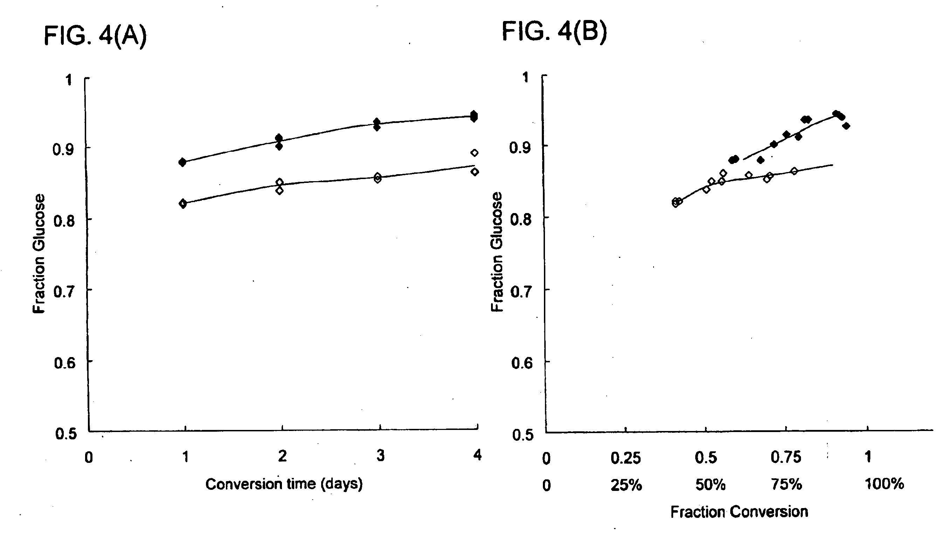

FIGS. 4A-B show the conversion of dilute acid treated corn stover to soluble sugars by 20 mg/g of whole cellulase from *Trichoderma reesei* over-expressing beta-glucosidase, at 38 °C (open symbols) and 53 °C (closed symbols).

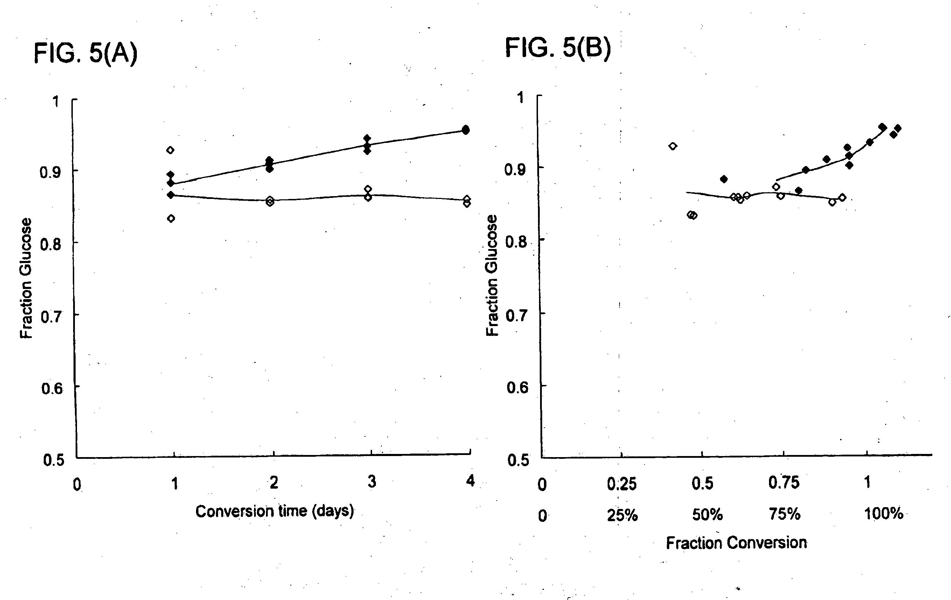

FIGS. 5A-B show the conversion of dilute acid treated corn stover to soluble sugars by 20 mg/g of whole cellulase from *Trichoderma reesei* over-expressing beta-glucosidase, at 38 °C (open symbols) and 53 °C (closed symbols).

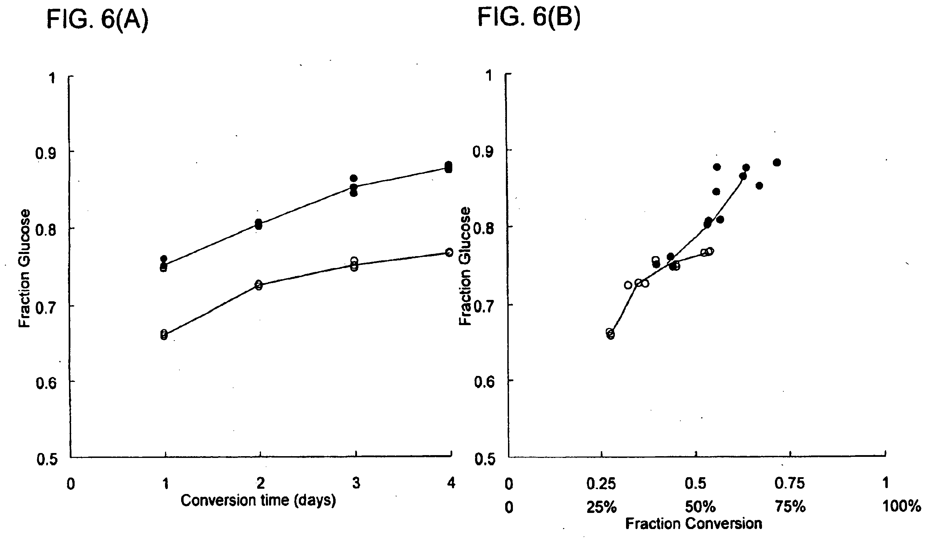

FIGS. 6A-B show the conversion of dilute acid treated corn stover to soluble sugars by 12 mg/g whole cellulase from *Trichoderma reesei,* at 38 °C (open symbols) and 53 °C (closed symbols).

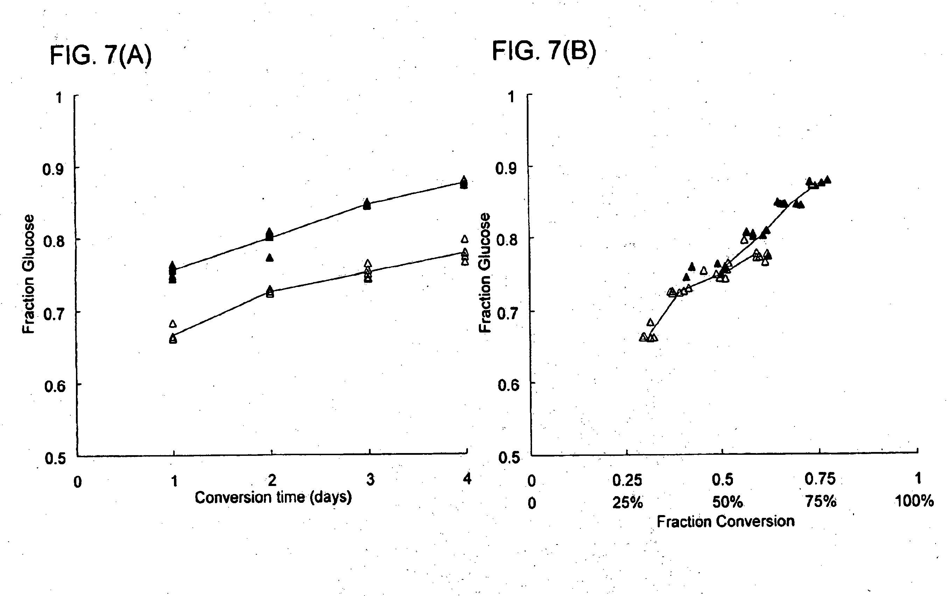

FIGS. 7A-B show the conversion of dilute acid treated corn stover to soluble sugars by 12 mg/g of whole cellulase from *Trichoderma reesei* expressing a CBH1-E1 fusion protein, 38 °C (open symbols) and 53 °C (closed symbols).

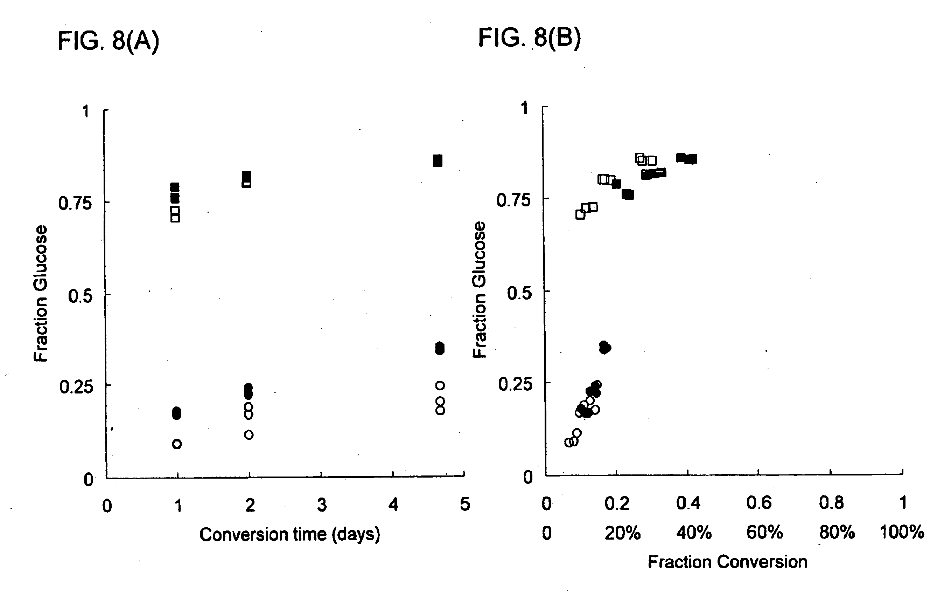

FIGS. 8A-B show the conversion of dilute acid treated corn stover to soluble sugars by 15 mg/g of an enzyme mixture of either EG1 and *T. reesei* CBH1 (squares) or E1 and *H. grisea* CBH1 (circles) at 38 °C (open symbols) and 65 °C (closed symbols).

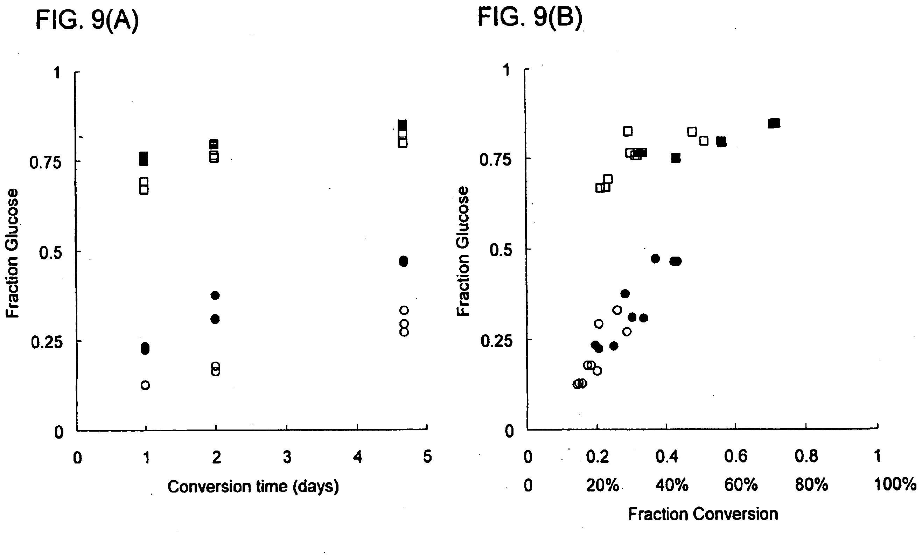

FIGS. 9A-B show the conversion of dilute acid treated corn stover to soluble sugars by 15 mg/g of an enzyme mixture of either EG1, *T. reesei* CBH1 and *T. reesei* CBH2 (squares) or E1, *H. grisea* CBH1 and *T. reesei* CBH2 (circles) at 38 °C (open symbols) and 65 °C (closed symbols).

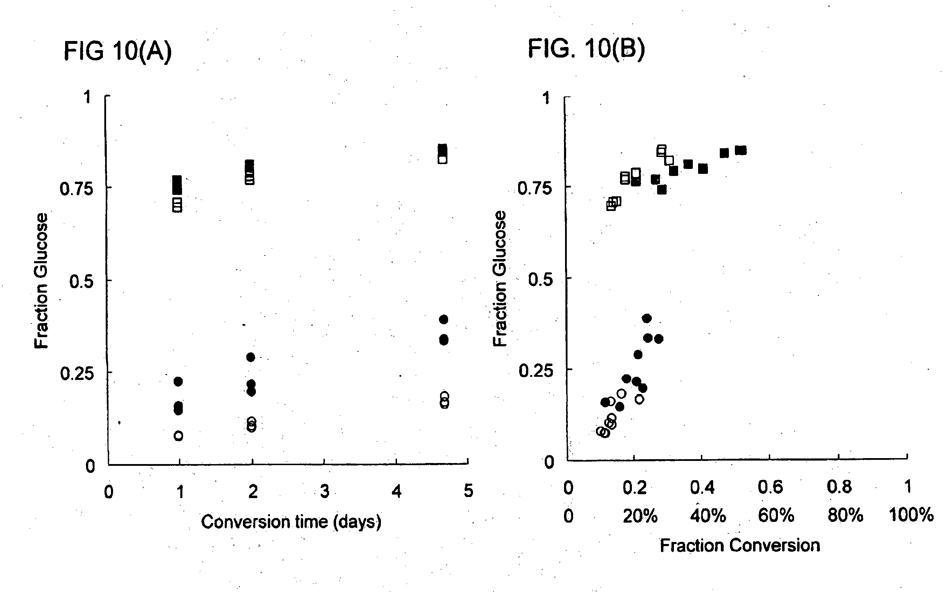

FIGS. 10A-B show the conversion of dilute acid treated corn stover to soluble sugars by 15 mg/g of an enzyme mixture of either EG1, *T. reesei* CBH1 (squares) and *T. fusca* E3 or E1, *H. grisea* CBH1 and *T. fusca* E3 (circles) at 38 °C (open symbols) and 65 °C (closed symbols).

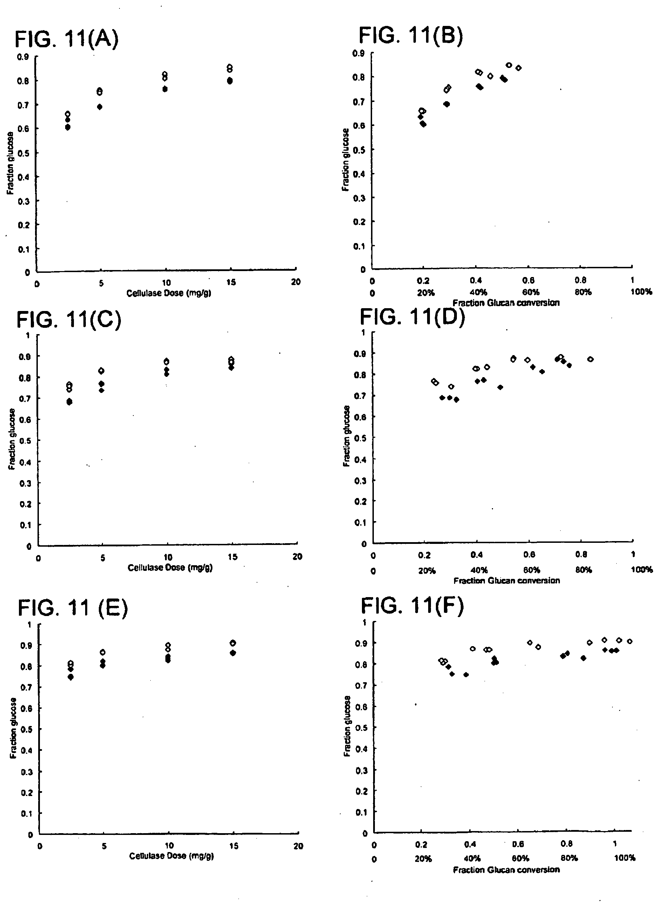

FIGS. 11A-F show the conversion of dilute acid treated corn stover to soluble sugars by a *Trichoderma reesei* strain at 53 °C (closed symbols) and 59 °C (open symbols)

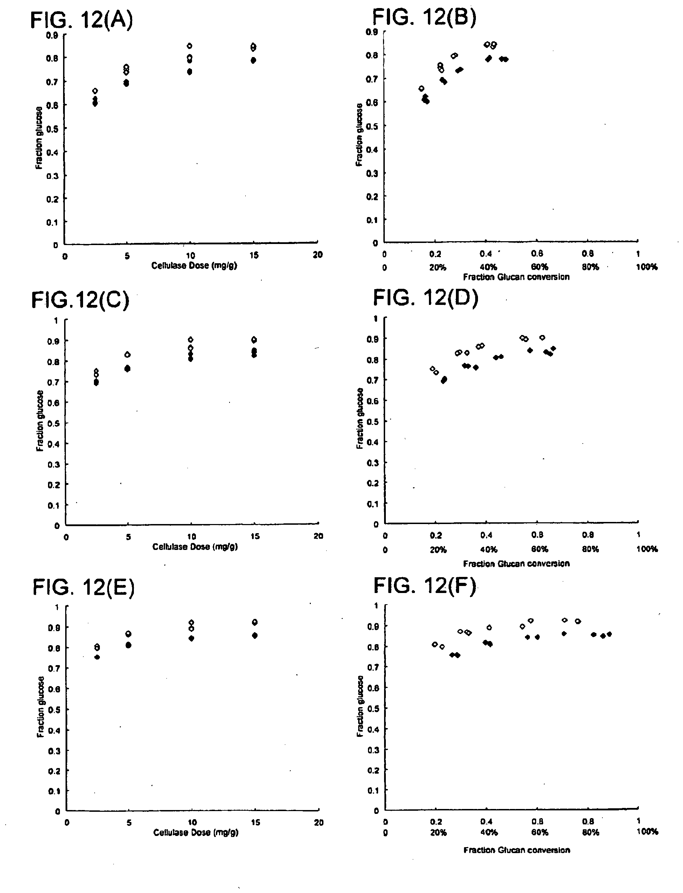

FIGS. 12A-F. The conversion of dilute acid treated corn stover to soluble sugars by a whole cellulase from *Trichoderma reesei* expressing a CBH 1-E1 fusion protein, at 53 °C (closed symbols) and 59 °C (open symbols).

### 7. DETAILED DESCRIPTION OF VARIOUS EMBODIMENTS

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, N.Y. (1991) provide one of skill with a general dictionary of many of the terms used in this invention. Although any methods and materials similar or equivalent to those described herein can be used in the practice or testing of the present invention, the preferred methods and materials are described. Numeric ranges are inclusive of the numbers defining the range. It is to be understood that this invention is not limited to the particular methodology, protocols, and reagents described, as these may vary.

The headings provided herein are not limitations of the various aspects or embodiments of the invention which can be had by reference to the specification as a whole. Accordingly, the terms defined immediately below are more fully defined by reference to the specification as a whole.

The term "cellulase" refers to a category of enzymes capable of hydrolyzing cellulose (beta-1,4-glucan or beta D-glucosidic linkages) polymers to shorter cello-oligosaccharide oligomers, cellobiose and/or glucose.

The term "exo-cellobiohydrolase" (CBH) refers to a group of cellulase enzymes classified as EC 3.2.1.91. These enzymes are also known as exoglucanases or cellobiohydrolases. CBH enzymes hydrolyze cellobiose from the reducing or non-reducing end of cellulose. In general a CBHI type enzyme preferentially hydrolyzes cellobiose from the reducing end of cellulose and a CBHII type enzyme preferentially hydrolyzes the non-reducing end of cellulose.

The term "cellobiohydrolase activity" is defined herein as a 1,4-D-glucan cellobiohydrolase (E.C. 3.2.1.91) activity which catalyzes the hydrolysis of 1,4-beta-D-glucosidic linkages in cellulose, cellotetriose, or any beta-1,4-linked glucose containing polymer, releasing cellobiose from the ends of the chain. For purposes of the present invention, cellobiohydrolase activity can be determined by release of water-soluble reducing sugar from cellulose as measured by the PHBAH method of Lever et al., 1972, Anal. Biochem. 47: 273-279. A distinction between the exoglucanase mode of attack of a cellobiohydrolase and the endoglucanase mode of attack can be made by a similar measurement of reducing sugar release from substituted cellulose such as carboxymethyl cellulose or hydroxyethyl cellulose (Ghose, 1987, Pure & Appl. Chem. 59: 257-268). A true cellobiohydrolase will have a very high ratio of activity on unsubstituted versus substituted cellulose (Bailey et al, 1993, Biotechnol. Appl. Biochem. 17: 65-76).

The term "endoglucanase" (EG) refers to a group of cellulase enzymes classified as EC 3.2.1.4. An EG enzyme hydrolyzes internal beta-1,4 glucosidic bonds of the cellulose. The term "endoglucanase" is defined herein as an endo-1,4-(1,3;1,4)-beta-D-glucan 4-glucanohydrolase (E.C. No. 3.2.1.4) which catalyses endohydrolysis of 1,4-beta-D-glycosidic linkages in cellulose, cellulose derivatives (for example, carboxy methyl cellulose), lichenin, beta-1,4 bonds in mixed beta-1,3 glucans such as cereal beta-D-glucans or xyloglucans, and other plant material containing cellulosic components. For purposes of the present invention, endoglucanase activity can be determined using carboxymethyl cellulose (CMC) hydrolysis according to the procedure of Ghose, 1987, Pure and Appl. Chem. 59: 257-268.

The term "beta-glucosidase" is defined herein as a beta-D-glucoside glucohydrolase (E.C. 3.2.1.21) which catalyzes the hydrolysis of cellobiose with the release of beta-D-glucose. For purposes of the present invention, beta-glucosidase activity may be measured by methods known in the art, e.g., HPLC.

"Cellulolytic activity" encompasses exoglucanase activity, endoglucanase activity or both types of enzyme activity, as well as beta-glucosidase activity.

Many microbes make enzymes that hydrolyze cellulose, including the bacteria *Acidothermus, Thermobifida, Bacillus,* and *Cellulomonas; Streptomyces;* yeast such as a *Candida, Kluyveromyces, Pichia, Saccharomyces, Schizosaccharomyces,* or *Yarrowia* and the fungi *Acremonium, Aspergillus, Aureobosidium, Chrysosporium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* or *Trichoderma,* or alternative sexual forms thereof such as *Emericella* and *Hypocrea* (See, Kuhls et al., 1996).

A "non-naturally occurring" composition encompasses those compositions produced by: (1) modifying an organism to overexpress or underexpress one or more cellulolytic enzyme; or (2) modifying an organism such that at least one cellulolytic enzyme is deleted or (3) modifying an organism to express a heterologous component cellulolytic enzyme.

We have found, in part, that increased saccharification temperature both increases the yield of glucose from cellulosic materials and also results in improved overall conversion of cellulose such that the fraction of glucose in the conversion product is increased at higher incubation temperatures.

The present teachings provide methods for increasing the yield of glucose from the enzymatic saccharification of cellulosic starting materials by incubating a cellulosic substrate or a pretreated cellulosic substrate with a Trichoderma reesei whole cellulase between 55°C and 65°C for 0.1 hours to 96 hour at 0 ptt of from 4 to 9.

In the methods of the present disclosure, the cellulosic material can be any cellulose containing material. The cellulosic material can include, but is not limited to, cellulose, hemicellulose, and lignocellulosic materials. In some embodiments, the cellulosic materials include, but are not limited to, biomass, herbaceous material, agricultural residues, forestry residues, municipal solid waste, waste paper, and pulp and paper residues. In some embodiments, the cellulosic material includes wood, wood pulp, papermaking sludge, paper pulp waste streams, particle board, corn stover, corn fiber, rice, paper and pulp processing waste, woody or herbaceous plants, fruit pulp, vegetable pulp, pumice, distillers grain, grasses, rice hulls, sugar cane bagasse, cotton, jute, hemp, flax, bamboo, sisal, abaca, straw, corn cobs, distillers grains, leaves, wheat straw, coconut hair, algae, switchgrass, and mixtures thereof (see, for example, Wiselogel et al., 1995, in Handbook on Bioethanol (Charles E. Wyman, editor), pp. 105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp. 23-40, Springer-Verlag, New York).

The cellulosic material can be used as is or may be subjected to pretreatment using methods known in the art. Such pretreatments include chemical, physical, and biological pretreatment. For example, physical pretreatment techniques can include without limitation various types of milling, crushing, steaming/steam explosion, irradiation and hydrothermolysis. Chemical pretreatment techniques can include without limitation dilute acid, alkaline, organic solvent, ammonia, sulfur dioxide, carbon dioxide, and pH-controlled hydrothermolysis. Biological pretreatment techniques can include without limitation applying lignin-solubilizing microorganisms. The pretreatment can occur from several minutes to several hours, such as from about 1 hour to about 120.

In one embodiment, the pretreatment may be by elevated temperature and the addition of either of dilute acid, concentrated acid or dilute alkali solution. The pretreatment solution can added for a time sufficient to at least partially hydrolyze the hemicellulose components and then neutralized

In some embodiments, the pretreatment is selected from a group consisting of steam explosion, pulping, grinding, acid hydrolysis, and combinations thereof.

The whole cellulase is reacted with the cellulosic material at a temperature between 55°C and 65°C. The pH is from pH 4, about pH 4.5, about pH 5, about pH 5.5, about pH 6, about pH 6.5, about pH 7, about pH 7.5, about pH 8.0, to about pH 8.5. Generally, the pH range will be from about pH 4.5 to pH 9. Incubation of the cellulase under these conditions results in release or liberation of substantial amounts of the soluble sugar from the cellulosic material. By substantial amount is intended at least 20%, 30%, 40%, 50%, 60%, 70%, 80%, 90%, 95% or more of soluble sugar is available sugar.

The cellulase treatment is from 0.1 hour to 96 hours, preferably about 12 hours to about 72 hours, more preferably about 24 to 48 hours.

The amount of cellulase is a function of the enzyme(s) applied and the reaction time and conditions given. Preferably, the cellulase(s) may be dosed in a total amount of from about 2 - 40 mg/g cellulosic material.

In the methods of the present disclosure, the cellulase is a whole cellulase. In some embodiments, the cellulase can be a whole cellulase preparation. As used herein, the phrase "whole cellulase preparation" refers to both naturally occurring and non-naturally occurring cellulase containing compositions. A "naturally occurring" composition is one produced by a naturally occurring source and which comprises one or more cellobiohydrolase-type, one or more endoglucanase-type, and one or more beta-glucosidase components wherein each of these components is found at the ratio produced by the source. A naturally occurring composition is one that is produced by an organism unmodified with respect to the cellulolytic enzymes such that the ratio of the component enzymes is unaltered from that produced by the native organism.

In general, the cellulases can include, but are not limited to: (i) endoglucanases (EG) or 1,4-β-d-glucan-4-glucanohydrolases (EC 3.2.1.4), (ii) exoglucanases, including 1,4-β-d-glucan glucanohydrolases (also known as cellodextrinases) (EC 3.2.1.74) and 1,4-β-d-glucan cellobiohydrolases (exo-cellobiohydrolases, CBH) (EC 3.2.1.91), and (iii) β-glucosidase (BG) or β-glucoside glucohydrolases (EC 3.2.1.21).

The whole cellulase is a Trichoderma reesei whole cellulase, such or RL-P37 (Sheir-Neiss et al., Appl. Microbiol. Biotechnology, 20 (1984), pp - 46-53 )

In some embodiments, the cellulase is a *Trichoderma reesei* RutC30 whole cellulase, which is available from the American Type Culture Collection as *Trichoderma reesei* ATCC 56765.

In the present disclosure, the cellulase can be from any cultivation method known in the art resulting in the expression of enzymes capable of hydrolyzing a cellulosic material. Fermentation can include shake flask cultivation, small- or large-scale fermentation, such as continuous, batch, fed-batch, or solid state fermentations in laboratory or industrial fermenters performed in a suitable medium and under conditions allowing the cellulase to be expressed or isolated.

Generally, the microorganism is cultivated in a cell culture medium suitable for production of enzymes capable of hydrolyzing a cellulosic material. The cultivation takes place in a suitable nutrient medium comprising carbon and nitrogen sources and inorganic salts, using procedures known in the art. Suitable culture media, temperature ranges and other conditions suitable for growth and cellulase production are known in the art. As a non-limiting example, the normal temperature range for the production of cellulases by *Trichoderma reesei* is 24°C to 28°C.

Certain fungi produce complete cellulase systems which include exo-cellobiohydrolases or CBH-type cellulases, endoglucanases or EG-type cellulases and beta-glucosidases or BG-type cellulases (Schulein, 1988). However, sometimes these systems lack CBH-type cellulases, e.g., bacterial cellulases also typically include little or no CBH-type cellulases. In addition, it has been shown that the EG components and CBH components synergistically interact to more efficiently degrade cellulose. See, e.g., Wood, 1985. The different components, i.e., the various endoglucanases and exocellobiohydrolases in a multi-component or complete cellulase system, generally have different properties, such as isoelectric point, molecular weight, degree of glycosylation, substrate specificity and enzymatic action patterns.

In some embodiments, the cellulase is used as is produced by fermentation with no or minimal recovery and/or purification. For example, once cellulases are secreted by a cell into the cell culture medium, the cell culture medium containing the cellulases can be used. In some embodiments the whole cellulase preparation comprises the unfractionated contents of fermentation material, including cell culture medium, extracellular enzymes and cells. Alternatively, the whole cellulase preparation can be processed by any convenient method, e.g., by precipitation, centrifugation, affinity, filtration or any other method known in the art. In some embodiments, the whole cellulase preparation can be concentrated, for example, and then used without further purification. In some embodiments the whole cellulase preparation comprises chemical agents that decrease cell viability or kills the cells. In some embodiments, the cells are lysed or permeabilized using methods known in the art.

A cellulase containing an enhanced amount of cellobiohydrolase and/or beta-glucosidase finds utility in ethanol production. Ethanol from this process can be further used as an octane enhancer or directly as a fuel in lieu of gasoline which is advantageous because ethanol as a fuel source is more environmentally friendly than petroleum derived products. It is known that the use of ethanol will improve air quality and possibly reduce local ozone levels and smog. Moreover, utilization of ethanol in lieu of gasoline can be of strategic importance in buffering the impact of sudden shifts in non-renewable energy and petrochemical supplies.

Ethanol can be produced via saccharification and fermentation processes from cellulosic biomass such as trees, herbaceous plants, municipal solid waste and agricultural and forestry residues. However, the ratio of individual cellulase enzymes within a naturally occurring cellulase mixture produced by a microbe may not be the most efficient for rapid conversion of cellulose in biomass to glucose. It is known that endoglucanases act to produce new cellulose chain ends which themselves are substrates for the action of cellobiohydrolases and thereby improve the efficiency of hydrolysis by the entire cellulase system. Therefore, the use of increased or optimized cellobiohydrolase activity may greatly enhance the production of ethanol.

Ethanol can be produced by enzymatic degradation of biomass and conversion of the released saccharides to ethanol. This kind of ethanol is often referred to as bioethanol or biofuel. It can be used as a fuel additive or extender in blends of from less than 1% and up to 100% (a fuel substitute).

Enhanced cellulose conversion may be achieved at higher temperatures using the CBH polypeptides described in, for example, any one of the following US Patent Publications US20050054039, US2005007459, US20060205042, US20050048619A1 and US20060218671. Methods of overexpressing beta-glucosidase are known in the art. See, for example, US 6,022,725. See also, for example, US20050214920.

The methods of the present disclosure can be used in the production of monosaccharides, disaccharides, and polysaccharides as chemical, fermentation feedstocks for microorganism, and inducers for the production of proteins, organic products, chemicals and fuels, plastics, and other products or intermediates. In particular, the value of processing residues (dried distillers grain, spent grains from brewing, sugarcane bagasse, etc.) can be increased by partial or complete solubilization of cellulose or hemicellulose. In addition to ethanol, some chemicals that can be produced from cellulose and hemicellulose include, acetone, acetate, glycine, lysine, organic acids (e.g., lactic acid), 1,3-propanediol, butanediol, glycerol, ethylene glycol, furfural, polyhydroxyalkanoates, cis, cis-muconic acid, animal feed and xylose.

The present teaching provide methods as disclosed above for converting a cellulosic material to glucose comprising combining a cellulosic material with a cellulase, incubating said cellulosic material and cellulase combination, cause a hydrolysis reaction to convert cellulosic material to soluble sugars, wherein the said soluble sugars comprises glucose and cellobiose and the fraction of glucose is at least 0.75 relative to said soluble sugars.

In some embodiments, the methods of the present disclosure further comprise the step of determining the amount of glucose and or soluble sugars.

Also provided are methods as diclosed above for converting a cellulosic material to glucose comprising the steps of combining a cellulosic material with a cellulase such that the resulting combination of cellulosic material and cellulase has 1% to about 30% cellulose by weight;

The present invention is described in further detail in the following examples which are not in any way intended to limit the scope of the invention as claimed. The attached Figures are meant to be considered as integral parts of the specification and description of the invention.

Aspects of the present teachings may be further understood in light of the following examples, which should not be construed as limiting the scope of the present teachings.

### 7. EXAMPLES

Cellulose conversion was evaluated by techniques known in the art. See, for example, Baker et al, Appl Biochem Biotechnol 70-72:395-403 (1998) and as described below. One hundred fifty microliters of substrate per well was loaded into a flat-bottom 96-well microtiter plate (MTP) using a repeater pipette. Twenty microliters of appropriately diluted enzyme solution was added on top. The plates were covered with aluminum plate sealers and placed in incubators at either test temperature, with shaking, for the times specified. The reaction was terminated by adding 100 µl 100 mM Glycine pH 10 to each well. With thorough mixing, the contents thereof were filtered through a Millipore 96-well filter plate (0.45 µm, PES). The filtrate was diluted into a plate containing 100 µl 10 mM Glycine pH 10 and the amount of soluble sugars produced measured by HPLC. The Agilent 1100 series HPLCs were all equipped with a deashing/guard column (Biorad #125-0118) and an Aminex lead based carbohydrate column (Aminex HPX-87P). The mobile phase was water with a 0.6 ml/min flow rate.

Pretreated corn stover (PCS) - Corn stover was pretreated with 2% w/w H₂SO₄ as described in Schell, D. et al., J. Appl. Biochem. Biotechnol. 105:69 - 86 (2003) and followed by multiple washes with deionized water to obtain a pH of 4.5. Sodium acetate was added to make a final concentration of 50mM and the solution was titrated to pH 5.0. The cellulose concentration in the reaction mixture was approximately 7%.

Using the following cellulases: *Trichoderma reesei* whole cellulase over-expressing beta-glucosidase 1 (WC - BGL1) (see for example, U.S. Patent No. 6,022,725, *Trichoderma reesei* whole cellulase expressing a CBH1-E1 fusion protein (WC - CBH1-E1) (see for example, US Patent Publication No. 20060057672), *Trichoderma reesei* Endoglucanase 1(EG1), *Trichoderma reesei* cellobiohydrolase 1 (CBH1) and *Trichoderma reesei* cellobiohydrolase 2 (CBH2), *Humicola grisea* cellobiohydrolase 1 (CBH1) and *Acidothermus cellulolyticus* endoglucanase E1 (E1), *Thermomonospera fusca* E3 exocellulase. The amount of enzyme was provided in milligrams per gram cellulose. The results of are summarized in FIGS 1-12. The ordinate represents the fraction of glucose with respect to the total sugar (wt/wt basis). For example, in FIG. 1-10, (A) the ordinate represents the length of conversion time and in FIG. 1-10, (B) the abscissa represents the total soluble sugar conversion that is observed (each incubation time is not explicitly labeled but a later incubation time is indicated by higher conversion) Figures 1 to 10 and the data corresponding to 53°C from Figures 11 and 12 are comparative examples.

FIGS. 1A-B show the conversion of dilute acid treated corn stover to soluble sugars by 3.3 mg/g whole cellulase from *Trichoderma reesei* over-expressing beta-glucosidase, at 38°C (open symbols) and 53 °C (closed symbols). *T. reesei* whole cellulase with elevated β-glucosidase levels converts acid-pretreated corn stover to a higher fraction of glucose at 53°C than at 38°C.

FIGS. 2A-B show the conversion of dilute acid treated corn stover to soluble sugars by 12 mg/g of whole cellulase from *Trichoderma reesei* over-expressing beta-glucosidase, at 38°C (open symbols) and 53 °C (closed symbols). *T. reesei* whole cellulase with elevated β-glucosidase levels converts acid-pretreated corn stover to a higher fraction of glucose at 53°C than at 38°C.

FIGS. 3A-B show the conversion of dilute acid treated corn stover to soluble sugars by 18 mg/g of whole cellulase from *Trichoderma reesei* over-expressing beta-glucosidase, at 38°C (open symbols) and 53 °C (closed symbols). *T. reesei* whole cellulase with elevated β-glucosidase levels converts acid-pretreated corn stover to a higher fraction of glucose at 53°C than at 38°C.

FIGS. 4A-B show the conversion of dilute acid treated corn stover to soluble sugars by 20 mg/g of whole cellulase from *Trichoderma reesei* over-expressing beta-glucosidase 1 at 38 °C (open symbols) and 53 °C (closed symbols). *T. reesei* whole cellulase with elevated β-glucosidase levels converts acid-pretreated corn stover to a higher fraction of glucose at 53°C than at 38°C.

FIGS. 5A-B show the conversion of dilute acid treated corn stover to soluble sugars by 25 mg/g whole cellulase from *Trichoderma reesei* over-expressing beta-glucosidase, at 38 °C (open symbols) and 53 °C (closed symbols). *T. reesei* whole cellulase with elevated β-glucosidase levels converts acid-pretreated corn stover to a higher fraction of glucose at 53°C than at 38°C.

FIGS. 6A-B show the conversion of dilute acid treated corn stover to soluble sugars by 12 mg/g of whole cellulase from *Trichoderma reesei,* at 38 °C (open symbols) and 53 °C (closed symbols). *T. reesei* whole cellulase converts acid-pretreated corn stover to a higher fraction of glucose at 53°C than at 38°C.

FIGS. 7A-B show the conversion of dilute acid treated corn stover to soluble sugars by 12 mg/g of whole cellulase from *Trichoderma reesei* expressing a CBH1-E1 fusion protein, at 38 °C (open symbols) and 53 °C (closed symbols). *T. reesei* whole cellulase converts acid-pretreated corn stover to a higher fraction of glucose at 53°C than at 38°C.

FIGS. 8A-B shows the conversion of dilute acid treated corn stover to soluble sugars by 15 mg/g of a mixture of cellulases composed of either *T. reesei* EG1 and *T. reesei* CBH1 (squares) orE1 and *H. grisea* CBH1 (circles) at 38 °C (open symbols) and 65 °C (closed symbols). Cellulase mixtures containing E1 convert acid-pretreated corn stover to a higher fraction of cellobiose than mixtures containing EG1.

FIGS. 9A-B show the conversion of dilute acid treated corn stover to soluble sugars by 15 mg/g of a mixture of cellulases composed of either EG1, *T. reesei* CBH1 and *T. reesei* CBH2 (squares) or E1, *H. grisea* CBH1 and *T. reesei* CBH2 (circles) at 38 °C (open symbols) and 65 °C (closed symbols). Cellulase mixtures containing E1 convert acid-pretreated corn stover to a higher fraction of cellobiose than mixtures containing EG1.

FIGS. 10A-B show the conversion of dilute acid treated corn stover to soluble sugars by 15 mg/g of a mixture of cellulases composed of either EG1. *T. reesei* CBH1 and *T. fusca* E3 (squares) or E1, *H. grisea* CBH1 and *T. fusca* E3 (circles) at 38 °C (open symbols) and 65 °C (closed symbols). Cellulase mixtures containing E1 convert acid-pretreated corn stover to a higher fraction of cellobiose than mixtures containing EG1.

FIGS. 11A-F show the conversion of dilute acid treated corn stover to soluble sugars by *Trichoderma reesei* whole cellulase at 53 °C (closed symbols) and 59 °C (open symbols) for 1 day (A and B), 2 days (C and D), and 3 days (E and F). The ordinate represents the fraction of glucose with respect to the total sugar (wt/wt basis) (A, B, and E). The abscissa represents the dose of enzyme used (B, D, and E). The abscissa represents the total soluble sugar conversion that is observed (each dose is not explicitly labeled, but a higher dose is indicated by higher conversion). *T. reesei* whole cellulase converts acid-pretreated corn stover to a higher fraction of glucose at high temperatures.

FIGS. 12A-F show the conversion of dilute acid treated corn stover to soluble sugars a *Trichoderma reesei* whole cellulase expressing a CBH1-E1 fusion protein, at 53 °C (closed symbols) and 59°C (open symbols) for (A and B) 1, (C and D) 2, and (E and F) 3 days. The ordinate represents the fraction of glucose with respect to the total sugar (wt/wt basis) (A, C, and E). The abscissa represents the dose of enzyme used (B, D, and F) The abscissa represents the total soluble sugar conversion that is observed (each dose is not explicitly labeled, but a higher dose is indicated by higher conversion), *T. reesei* whole □ ellulose converts acid-pretreated corn stover to a higher fraction of glucose at high temperatures.

It is understood that the examples and embodiments described herein are for illustrative purposes only

## Claims

1. A method for converting a cellulosic material to glucose comprising the steps of:
combining a cellulosic material with a *Trichoderma reesei* whole cellulase such that the resulting combination of cellulosic material and cellulase has 1% to 30% cellulose by weight; and
incubating said cellulosic material and cellulase combination at a temperature between 55 °C and 65 °C for 0.1 hours to 96 hours at a pH of from 4 to 9 to cause a hydrolysis reaction to convert at least 20% of said cellulosic material to soluble sugars,
wherein said soluble sugars comprises glucose and cellobiose, and the fraction of glucose is at least 0.75 relative to said soluble sugars
and the yield of glucose increases at incubating temperatures above 50°C compared to the yield of glucose at an incubating temperature of 38°C.

2. The method of Claim 1 wherein said Trichoderma reesei expresses a recombinant enzyme.

3. The method of Claim 2 wherein said recombinant enzyme is a beta-glucosidase.

4. The method any one of the preceding claims, wherein the cellulosic material selected from the group consisting of bioenergy crops, agricultural residues, municipal solid waste, industrial solid waste, yard waste, wood, forestry waste, switchgrass, waste paper, sludge from paper manufacture, corn grain, corn cobs, corn husks, corn stover, grasses, wheat, wheat straw, hay, rice straw, sugar cane bagasse, sorghum, soy, trees, switchgrass, hay, barley, barley straw, rice straw, and grasses.

5. The method of any one of the preceding claims, further comprising pretreating said cellulosic material.

6. The method of Claim 5 wherein said pretreatment is selected from a group consisting of steam explosion, pulping, grinding, acid hydrolysis, and combinations thereof.

7. The method of any one of the preceding claims, further comprising determining the amount of glucose.

8. The method of any one of the preceding claims, further comprising determining the amount of soluble sugars.

9. The method of any one of the preceding claims, wherein the amount of said cellulase mixture is about 2 mg - 40 mg/g cellulosic material.

## Patentansprüche

1. Verfahren zur Überführung eines celluloseartigen Materials in Glucose, das die folgenden Schritte umfasst:
das Kombinieren eines celluloseartigen Materials mit einer intakten *Trichoderma-reesei-*Cellulase, so dass die resultierende Kombination aus celluloseartigem Material und Cellulase 1 bis 30 Gew.-% Cellulose aufweist, und
0,1 h bis 96 h langes Inkubieren der Kombination aus celluloseartigem Material und Cellulase bei einer Temperatur zwischen 55 °C und 65 °C und einem pH-Wert von 4 bis 9, um eine Hydrolysereaktion herbeizuführen und so zumindest 20 % des celluloseartigen Materials in lösliche Zucker überzuführen,
wobei die löslichen Zucker Glucose und Cellobiose umfassen, der Anteil an Glucose zumindest 0,75 in Bezug auf die löslichen Zucker ausmacht und die Glucoseausbeute bei Inkubationstemperaturen von über 50 °C im Vergleich zu der Glucoseausbeute bei einer Inkubationstemperatur von 38 °C ansteigt.

2. Verfahren nach Anspruch 1, wobei die *Trichoderma reesei* ein rekombinantes Enzym exprimiert.

3. Verfahren nach Anspruch 2, wobei das rekombinante Enzym eine β-Glucosidase ist.

4. Verfahren nach einem der vorangegangenen Ansprüche, wobei das celluloseartige Material aus der aus Bioenergiegetreide, landwirtschaftlichen Überresten, kommunalem Festabfall, gewerblichem Festabfall, Gartenabfall, Holz, Waldabfall, Rutenhirse, Papiermüll, Schlamm aus der Papierherstellung, Maiskorn, Maiskolben, Maisschoten, Maisstroh, Gräsern, Weizen, Weizenstroh, Heu, Reisstroh, Zuckerrohrbagasse, Sorghumhirse, Soja, Bäumen, Rutenhirse, Heu, Gerste, Gerstenstroh, Reisstroh und Gräsern bestehenden Gruppe ausgewählt ist.

5. Verfahren nach einem der vorangegangenen Ansprüche, das weiters das Vorbehandeln des celluloseartigen Materials umfasst.

6. Verfahren nach Anspruch 5, wobei die Vorbehandlung aus der aus Dampfexplosion, Aufschluss, Mahlen, Säurehydrolyse und Kombinationen davon bestehenden Gruppe ausgewählt ist.

7. Verfahren nach einem der vorangegangenen Ansprüche, das weiters das Bestimmen der Menge an Glucose umfasst.

8. Verfahren nach einem der vorangegangenen Ansprüche, das weiters das Bestimmen der Menge an löslichen Zuckern umfasst.

9. Verfahren nach einem der vorangegangenen Ansprüche, wobei die Menge des Cellulasegemischs etwa 2 bis 40 mg/g celluloseartiges Material beträgt.

## Revendications

1. Procédé pour convertir un matériau cellulosique en glucose comprenant les étapes de:
combiner un matériau cellulosique avec une cellulase entière de *Trichoderma reesei* de telle sorte que la combinaison obtenue du matériau cellulosique et de la cellulase présente 1% à 30% de cellulose en poids; et
incuber ladite combinaison de matériau cellulosique et de cellulase à une température entre 55°C et 65°C pendant 0,1 heure à 96 heures à un pH de 4 à 9 pour provoquer une réaction d'hydrolyse afin de convertir au moins 20% dudit matériau cellulosique en sucres solubles,
où lesdits sucres solubles comprennent du glucose et du cellobiose, et la fraction de glucose est au moins de 0,75 relativement auxdits sucres solubles, et le rendement de glucose augmente à des températures d'incubation supérieures à 50°C en comparaison avec le rendement du glucose à une température d'incubation de 38°C.

2. Procédé selon la revendication 1, où ledit *Trichoderma reesei* exprime une enzyme recombinante.

3. Procédé selon la revendication 2, où ladite enzyme recombinante est une béta-glucosidase.

4. Procédé selon l'une quelconque des revendications précédentes, où le matériau cellulosique est sélectionné dans le groupe consistant en récoltes bioénergétiques, résidus d'agriculture, déchets solides municipaux, déchets solides industriels, résidus de jardinage, bois, résidus forestiers, le panic raide, déchets de papier, boues de la fabrication de papier, grains de maïs, épis de maïs, enveloppes de maïs, tiges de maïs, herbes, blé, paille de blé, foin, la paille de riz, bagasse de canne à sucre, sorgho, soja, arbres, panic raide, foin, orge, paille d'orge, paille de riz et herbes.

5. Procédé selon l'une quelconque des revendications précédentes, comprenant en outre le prétraitement dudit matériau cellulosique.

6. Procédé selon la revendication 5, où le prétraitement est sélectionné dans un groupe consistant en explosion de vapeur, réduction en pâte, meulage, hydrolyse acide et leurs combinaisons.

7. Procédé selon l'une quelconque des revendications précédentes, comprenant en outre la détermination de la quantité de glucose.

8. Procédé selon l'une quelconque des revendications précédentes, comprenant en outre la détermination de la quantité de sucres solubles.

9. Procédé selon l'une quelconque des revendications précédentes, où la quantité dudit mélange de cellulase est d'environ 2 mg - 40 mg/g de matériau cellulosique.
